# EUROPEAN PATENT APPLICATION

(11) **EP 1 348 405 A1**
(43) Date of publication of application: **01.10.2003**
(21) Application number: 03447061.7
(22) Date of filing: 25.03.2003
(51) Int. Cl.: A61F 2/06

(54) **Improved intraluminar perforated radially expandable drug delivery prosthesis**

(30) Priority: 29.03.2002 EP 02447053
(71) Applicant: Advanced Laser Applications Holding S.A., 1637 Luxembourg (LU)
(72) Inventor: Sohier, Jürgen, 3000 Leuven (BE)
(74) Representative: Van Reet, Joseph

(57) **Abstract**

The radially expandable prosthesis for implantation in a lumen shows an inner and an outer surface and comprises a tubular wall composed of elongated struts (1, 2, 4) with a predetermined strut width. The struts are arranged to enable an expansion from a non-expanded state of the prosthesis to an expanded state. At least a number of the struts further show at least one location (5) provided with at least one hole (6) at the outer surface of the prosthesis. In order to maintain the required radial and fatigue strength of the prosthesis when providing the holes (6) without reducing the flexibility of the prosthesis and without increasing the additional amount of material therefor to a too large extend, the struts have at said locations an increased width (W₂) larger than the predetermined width (W₁) of the strut before and/or after said location (5).

## Description

The present invention relates to a radially expandable prosthesis or stent for implantation in a lumen, showing an inner and an outer surface and comprising a tubular wall composed of elongated struts with a predetermined strut width and arranged to enable an expansion from a non-expanded state of the prosthesis to an expanded state, the prosthesis having an outer surface provided with holes.

In practice, intraluminal prostheses or stents are generally known. They can be implanted in a lumen, for example an artery, to strengthen, support or repair the lumen. With coronary balloon dilatation for example, often a prosthesis is implanted in the place where a coronary artery is injured or where it tends to collapse. Once implanted, the prosthesis strengthens that part of the artery in a way the blood flow is ensured. A prosthesis configuration which is extremely suited for implantation in a body lumen, is a generally cylindrical prosthesis which can radially expand from a first small diameter to a second larger one. Such prostheses can be implanted in the artery by placing them on a catheter and transporting them through the artery to the desired location. The catheter is provided with a balloon or another expansion mechanism which exerts a radial outwards pressure on the prosthesis so that the prosthesis expands to a larger diameter. These prostheses are sufficiently strong to stay in shape after expansion, even after removal of the catheter.

Radially expandable prostheses are available in a variety of configurations, in this way an optimal efficacy is ensured in different particular situations. The patents of Lau (US Patent Nos. 5,514,154, 5,421,955, and 5,242,399), Baracci (US Patent No. 5,531,741), Gaterud (US Patent No. 85,522,882), Gianturco (US Patent Nos. 5,507,771 and 5,314,444), Termin (US Patent No. 5,496,277), Lane (US Patent No. 5,494,029), Maeda (US Patent No. 5,507,767), Marin (US Patent No. 5,443,477), Khosravi (US Patent No. 5,441,515), Jessen (US Patent No. 5,425,739), Hickle (US Patent No. 5,139,480), Schatz (US Patent No. 5,195,984), Fordenbacher (US Patent No. 5,549,662) and Wiktor (US Patent No. 5,133,732) all contain a sort of radially expandable prosthesis for implantation in a body lumen.

A major problem of the above mentioned intraluminal prostheses is the insufficient biocompatibility of these prostheses, when they are implanted intravascularly. They can cause acute or subacute thrombotic occlusions due to thrombus formation resulting in a considerable morbidity and even mortality. Furthermore these prostheses evoke a foreign body reaction with a considerable inflammation all around the prosthesis inducing fibromuscular cellular proliferation and narrowing of the prosthesis.

In order to reduce these problems it is already known to coat the prosthesis with a therapeutic agent or medicine increasing the biocompatibility of the prosthesis. EP-A-0 950 386 and WO-A-01/66036 disclose moreover to provide reservoirs or holes in the outer surface of the prosthesis. These reservoirs are filled with the therapeutic agent or with the medicine showing an anti-thrombotic and/or anti-restenotic action. By providing holes or reservoirs, the period of time over which the prosthesis can release an effective amount of these substances is considerably prolonged. The holes or reservoirs are filled in particular with a medicine suppressing the foreign body reaction against the prosthesis increasing thereby also the biocompatibility of the prosthesis.

The present inventors have however found that the presence of reservoirs or holes in the struts of the prostheses disclosed in EP-A-0 950 386 and WO-A-01/66036 reduces the radial strength and the fatigue strength or durability of the prosthesis so that, with the known prosthesis configurations, the thickness and/or the width of the struts has to be increased to maintain the required strength when applying reservoirs or holes in these struts. These measures involve however important drawbacks.

First of all, when implanted in the body lumen, the larger amount of metal will cause a greater foreign body reaction and therefore more neointimal hyperplasia resulting in a greater risk of re-occlusion of the lumen. Also the higher rigidity of the prosthesis can invoke more damages and cell proliferation in the lumen as a result of friction between the prosthesis and the inner wall of the lumen.

Before being implanted into the body lumen, i.e. in their non-expanded state, the prostheses are moreover less flexible, show a reduced crimpability (i.e. the ability to be crimped to a smaller diameter before implantation) and have a central axis that remains rather linear. Due to such a reduced flexibility the insertion of the prosthesis in the artery to be correctly placed in the body lumen is hampered. Another problem is the more pronounced decrease in axial length at radial expansion when the struts of the prosthesis have a larger width and/or thickness. When a prosthesis is placed in the artery or in another body lumen, the implantation has to be performed precisely in the desired place. Intraluminal prostheses are often exactly placed before their expansion, but due to the expansion the axial shortening causes that the prosthesis finally does not turn up in the correct place. Another increased problem is the occlusion of side branches. In the case of coronary arteries this can cause a myocardial infarction.

An object of the present invention is therefore to provide a new prosthesis, the struts of which are provided with holes, but which nevertheless can be given the required radial and fatigue strength without reducing the flexibility and/or crimpability of the prosthesis and increasing the additional amount of material required to maintain the required radial and fatigue strength to a too large extent.

For this purpose, the prosthesis according to the invention is characterised in that at least a number of said struts show at least one location which is provided with at least one of said holes and at which the strut has an increased width larger than the predetermined width of the strut before and/or after said location.

According to the invention it has been found that when providing holes in the struts of a prosthesis, the required radial and fatigue strength can be maintained by increasing the width of the struts only at the location of the holes and that, compared to a general increase in strut width, such local widenings of the struts have a smaller effect on the flexibility and/or crimpability of the prosthesis, especially in the non-expanded state thereof. To minimise the effect on the flexibility and/or crimpability of the prosthesis, at least a number of the locations with the holes are provided with one single hole, i.e. when a strut comprises more holes it shows a number of locations with an increased width corresponding to the number of holes. In successive struts, these locations can be longitudinally displaced with respect to one another so that, after cutting, the prosthesis can be crimped to a smaller diameter to facilitate the implantation thereof.

In a preferred embodiment of the prosthesis according to the invention, at least a number of the struts which show at least one of said locations have such a thickness that the ratio of the strut width before and/or after said locations over the strut thickness is greater than 0.5, and preferably greater than 0.6. Preferably all the struts have such a width and a thickness that the ratio of the strut width over the strut thickness is everywhere greater than 0.5, and preferably greater than 0.6, in particular also at the transition of the different struts. In other words the prosthesis is free of so-called ductile hinges. An advantage of this embodiment is that the prosthesis has an increased durability since ductile hinges form weak spots. Due to the fact that in the prosthesis according to the invention the flexibility and/or crimpability can be maintained or is less reduced, the presence of such weak spots can be avoided.

In an advantageous embodiment of the prosthesis according to the invention, the prosthesis comprises at least two mutually connected circumferential sets of struts each comprising an alternating succession of longitudinal struts, extending in a general longitudinal direction, and transverse struts, extending in a generally circumferential direction and interconnecting two successive longitudinal struts, at least a number of said longitudinal struts showing at least one of said locations provided with at least one hole, the transverse struts being preferably free of said locations.

An advantage of this embodiment is that by providing the locations with the holes on the longitudinal struts, the flexibility of the prosthesis is less reduced by the widenings of the struts at the location of the holes.

In a preferred embodiment of the prosthesis according to the invention, said hole is a non-perforating hole showing a depth smaller than the thickness of the strut or a perforating hole showing a depth equal to the thickness of the strut, the hole having an average width measured over the depth of the hole, in a direction perpendicular to the longitudinal direction of the strut, and an average length measured over the depth of the hole, in the longitudinal direction of the strut, which comprises at the most five times, preferably at the most three times, the average hole width, the average hole length being most preferably substantially equal to the average hole width.

Since the length of the holes comprises at the most five times the width thereof, more holes can be provided in the outer surface of the prosthesis, i.e. at shorter mutual distances, so that a more homogenous drug delivery is possible, compared for example to the prosthesis disclosed in EP-A-0 950 386 wherein the holes or reservoirs are formed by relatively shallow channels. A further advantage of such shorter holes is that they can be made deeper without affecting the required radial strength and durability of the prosthesis. In the prosthesis according to the invention, the holes extend indeed preferably over a depth in the struts which is greater than 30%, preferably greater than 50% and most preferably greater than 60% of the thickness of the strut. In this way, it is possible to incorporate more therapeutic agent in the prosthesis and to increase the release period thereof due to the fact that a larger amount of therapeutic agent can be contained in one hole relative to the surface area of the outer opening thereof in the outer surface of the prosthesis through which the therapeutic agent is released. The small holes, which may show a bottom or extend entirely through the strut wherein they are made, allow to load the prosthesis with a dose of medicine up to a thousand times higher compared to a non-perforated prosthesis. In this way a more biocompatible intraluminal prosthesis can be obtained which can also be used as a vehiculum for releasing and/or depositing medicines locally.

In a preferred embodiment, at least a bottom portion of said hole is substantially conical, the hole having either a bottom or extending through the strut forming in said inner surface of the tubular wall an inner opening.

An important advantage of this embodiment is that the holes can be made easily by laser cutting, in particular in accordance with the liquid guided laser cutting technique disclosed for example in US-A-5 902 499, by simply directing the laser beam to the desired spot and cutting the hole without any further movement of the laser beam. The depth of the hole can then simply be controlled by adjusting the total amount of energy of the laser beam, i.e. the pulse width, the duration and the intensity thereof. When making perforating holes, the diameter of the inner opening of the holes on the inner side of the strut can be controlled in the same way, i.e. also by adjusting the amount of energy used to make the hole by means of the laser beam. In other words, the amount of therapeutic agent released towards the inside of the prosthesis can be easily controlled by selecting the desired diameter of the inner openings. The total amount of cutting energy can be increased until the inner opening is substantially as large as the outer opening.

Other particularities and advantages of the invention will become apparent from the following description of some particular embodiments of the method and the prosthesis according to the present invention. The reference numerals used in this description relate to the annexed drawings wherein:
Figure 1 is a top plan view on a tubular prosthesis which has been cut in its longitudinal direction and pressed into a flat sheet;
Figure 2 shows on a larger scale a portion of the sheet illustrated in Figure 1;
Figure 3 is a view similar to the view of Figure 1 but showing another embodiment of the invention; and
Figure 4 shows, on a larger scale, a schematic cross-sectional view along lines IV-IV in Figure 2, illustrating a perforating hole with a substantially cylindrical shape.

In general the present invention relates to radially expandable prostheses for implantation in a lumen which comprise a tubular wall produced from sheet metal wherein the configuration of the prosthesis is cut out for example by means of a laser beam which is preferably guided in a water jet as disclosed in WO-A-01/66036. Instead of starting from a tubular member, use could also be made of a flat sheet which is enrolled and welded together to form the tubular prosthesis. When, after having cut the prosthesis and the holes, the prosthesis is polished, in particular by an electropolishing process, the thickness T of the prosthesis is somewhat smaller than the tickness of the tubular member or of the flat sheet. Usually the thickness T of the prosthesis is comprised between 50 and 200 µm, more particularly between 75 and 150 µm. In the following examples, the wall thickness T comprises for example about 125 µm. This thickness is achieved after an electropolishing process starting from a tubular member having a wall thickness of about 150 µm.

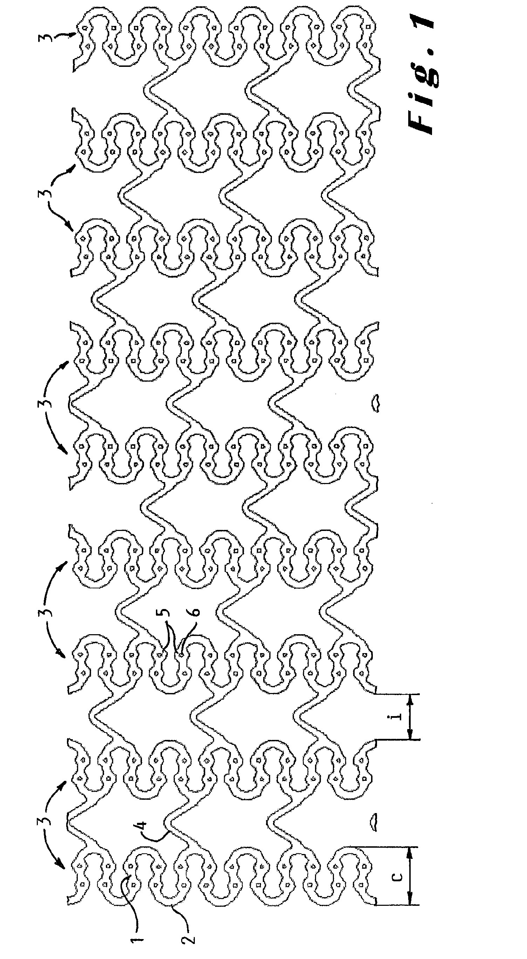

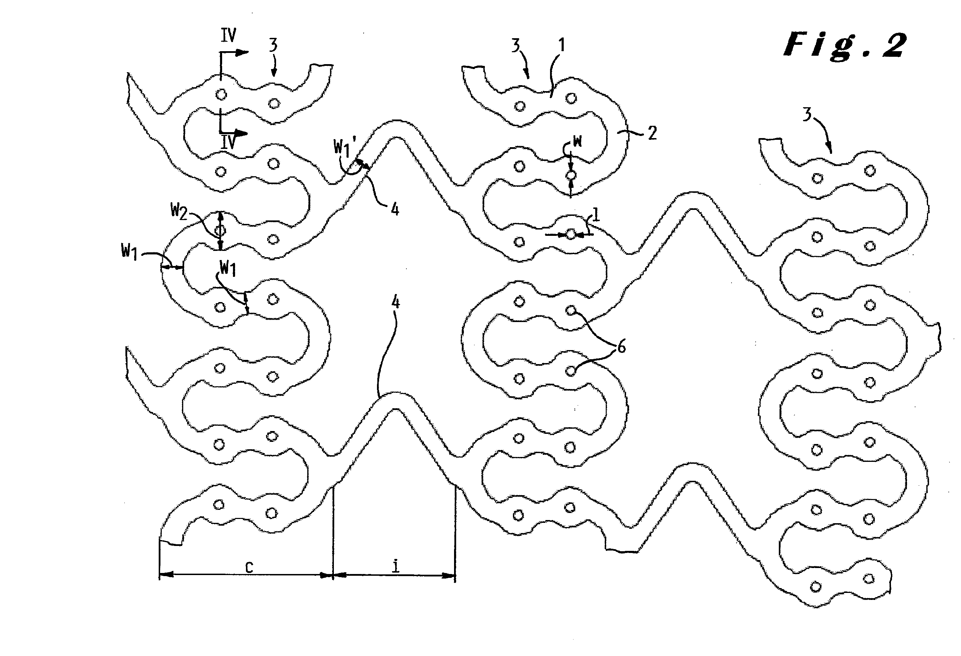

Figures 1 and 2 illustrate a first embodiment of a radially expandable prosthesis that presents little or none axial shortening at radial expansion. The tubular or more particularly cylindrical wall of this prosthesis is composed of struts with a predetermined strut width W₁ which are arranged to enable an expansion of the prosthesis from a non-expanded state, illustrated in the Figures, to an expanded state.

To provide the necessary radial support of the lumen, the struts comprise longitudinal struts 1, extending in a general longitudinal or axial direction of the prosthesis, and transverse struts 2, extending in a generally circumferential direction of the prosthesis. These longitudinal and transverse struts form at least two filaments or circumferential sets 3 of struts each comprising an alternating succession of longitudinal 1 and transverse struts 2, the transverse struts 2 interconnecting two successive longitudinal struts 1 and the longitudinal struts 1 interconnecting two successive transverse struts 2. The transverse struts 2 are preferably curved over an angle of at least 120°, and more preferably over an angle of at least 140°.

The prosthesis can exist of a variable amount of filaments or circumferential sets 3 of struts 1 and 2 which all constitute the prosthesis and describe in particular the outline of a cylindrical contour. At least two filaments 3 are necessary, including a first and a second ending filament to determine the extremities of the prosthesis contour. In the embodiment of Figure 1, the prosthesis comprises nine filaments. These filaments 3 all show a waving contour in the shape of consecutive omegas. Consequently each filament is composed of a number of turns with lowest points and tops zigzag crossing over the length of each filament. The lowest point is the most distant from the adjacent filament and the top is the most closely situated to the adjacent filament. Figure 1 shows a typical configuration with 12 turns, a number that can vary from 3 to 36 turns. The size of each filament 3, provided as the distance c between lowest point and top, changes when the prosthesis expands radially, mostly the size diminishes. In Figure 1 a typical configuration is shown with a distance c of about 1.0 mm between the lowest point and top, this distance however can vary from 0.5 to 5 mm or even within larger limits.

The end filaments are attached to adjacent intermediate filaments by means of undulating connecting struts 4 that act as axial elements joining two adjacent filaments. The illustrated connecting struts 4 are generally V-shaped but may for example also present the shape of an omega. The connecting struts 4 are also able to fasten together intermediate filaments. Each connecting strut 4 is attached to the adjacent filaments with a first connection point to the one end of the connecting piece and a second one to the other end. Both connecting points are situated in the tops of the filaments. Thus the connecting points are bridging the distance/opening between adjacent filaments with the interstice i as maximal width. In a variant embodiment, the connecting struts 4 may however also connect the tops of one filament with a bottom within the adjacent filament as disclosed for example in EP-A-0 931 520. Instead of being attached to the transverse struts 2, the connecting struts 4 may also be attached to the longitudinal struts 1.

In the embodiment illustrated in the Figures, this interstice i comprises about 0.75 mm resulting in a total length of the prosthesis of about 15 mm. Not necessarily all perforations are bridged with axial connecting parts. Separate outlined intermediate elements can be joined together by means of junctions that are connected with the intermediate elements on locations distant of the lowest points. Depending on the flexibility needs of the prosthesis a variable number of tops can be provided with connecting parts that link adjacent filaments. In case a higher flexibility is necessary, more tops will stay empty with at the minimum only one connecting piece between two adjacent filaments. The prosthesis is constructed such that during gradual expansion of the prosthesis the filament waves will in a first phase become somewhat larger and than gradually become shorter. To compensate for this shortening the V or omega shaped interconnections will gradually enlarge resulting in a less axial shortening during gradual expansion.

The above described configuration of the illustrated prosthesis is only given as an example and the basic principle of the invention may be applied to many different prosthesis designs. An essential feature of the present invention is that at least a number of the struts of the prosthesis show at least one location 5 which is provided with at least one hole 6 at the outer surface of the prosthesis and that, at that location 5, the strut has an increased width W₂, larger than the strut width W₁ before and/or after the location 5. The strut width W₁ may be different for the different types of struts, i.e. for the longitudinal struts 1, the transverse struts 2 and the connecting struts 4. Moreover, when a strut is provided with a series of two or more different locations with an increased width, the width of the strut between the successive locations does not have to be equal to the strut width before or after the series of locations but may in particular somewhat larger, for example about 150 µm when the strut width before and after the series of locations is for example about 120 µm. Since in the illustrated embodiment the longitudinal and the transverse struts have a same main function, namely the function of providing the necessary radial support to the wall of the lumen wherein the prosthesis will be implanted by forming the circumferential filaments 3, they have a same width W₁, more particularly a width of for example about 130 µm. The main function of the connecting struts 4 is however not to provide a radial support but to provide a rather flexible connection between the filaments 3. In the embodiment illustrated in the figures, these connecting struts were given therefore a smaller width W'₁, in particular a width of about 100 µm.

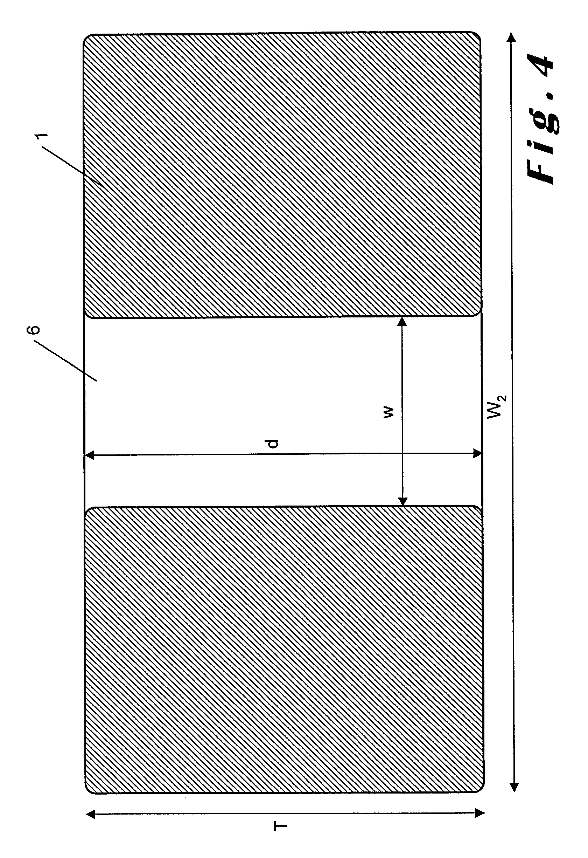

The holes 6 may be perforating holes or perforations, having a depth d equal to the thickness T of the strut or they may be non-perforating holes or pits having a depth d smaller than the thickness T of the strut and enabling to obtain a directional release of the therapeutic agent contained in the hole. In Figure 4 only a perforating hole has been illustrated. Other types of holes including conical perforations, conical pits and perforations formed by a cylindrical top portion followed by a conical bottom portion are illustrated in Figures 9 to 13 of WO-A-01/66036 which are taken up herein by way of reference. All of these holes have an average width w measured over the depth of the hole, in a direction perpendicular to the longitudinal or axial direction of the strut, and an average length I also measured over the depth of the hole but in the longitudinal or axial direction of the strut. For straight holes, in particular for cylindrical holes as illustrated in Figure 4 the average length and width corresponds of course to the actual length I and width w. In the embodiment illustrated in the figures, both the length I and the width w of the cylindrical holes 6 comprises about 60 µm. Such cylindrical holes, or even conical holes or holes showing a conical bottom, can easily be made by laser cutting, in particular by means of water-guided laser technology.

In general, the average length I of the hole 6 should preferably comprise at the most five times, and preferably at the most three times, the average width w thereof whilst the hole 4 itself should preferably extend over a depth d in the strut which is larger than 30%, preferably larger than 50%, and most preferably larger than 60%, of the thickness T of the strut 1. In this way, the therapeutic agent is distributed over a number of relatively small holes enabling a homogeneous distribution thereof over the surface of the prosthesis. The total amount of therapeutic agent applied onto the prosthesis can be controlled not only by the number of holes but also by the depth thereof. An advantage of providing deeper holes is that the surface of the opening through which the therapeutic agent can be released out of the hole is relatively small compared to the volume of the hole so that the duration of the therapeutic agent release can be extended.

The holes 6 have advantageously an average width w larger than 10 µm, in particular larger than 20 µm and more particularly larger than 30 µm but smaller than 130 µm, preferably smaller than 90 µm and most preferably smaller or equal to 80 µm. The average length I of the holes 6 may comprise up to five times this width w but is preferably substantially equal to the width w. As explained hereabove, the holes 6 are in particular preferably substantially cylindrical.

In a preferred embodiment, the average width w of the holes 6 comprises at the most 70%, preferably at the most 60%, of the width W₁ of the strut. Together with the limited average length I of the holes 6 this relatively small width enables to increase the depth d of the holes (until a perforating hole is achieved) with a minimum increase of the width at the locations 5 of the holes 6 and thus with a minimum additional amount of prosthesis material and a minimum effect on the flexibility of the prosthesis in its unexpanded state.

In a preferred embodiment of the invention, the increased width W₂ of the struts at the location 5 of the holes 6 is at least 5%, preferably at least 20% and more preferably at least 50% larger than the width W₁ before and/or after this location. When the hole 6 is a perforating hole, the increased width W₂ at the location 5 of this hole 6 is preferably at least equal to the sum of the strut width W₁ before and/or after this location and the average width w of the perforating hole, and is more preferably at least equal to the sum of the strut width W₁ and 1.5 times the averaged hole width w.

In the example illustrated in Figures 1 and 2, each longitudinal strut 1 is provided with two perforating holes 6 having an average width w and length I of about 60 µm. At the locations 5 of these holes 6, the struts have a width W₂ of about 250 µm whereas before and after these locations the struts have a width W₁ of about 130 µm. In contrast to the longitudinal struts 1, the transverse struts 2 are not provided with holes or with widenings so that the strength and especially the flexibility of these struts is maintained. In fact in the illustrated embodiment these transverse struts must enable the transition from the non-expanded to the radially expanded state of the prosthesis.

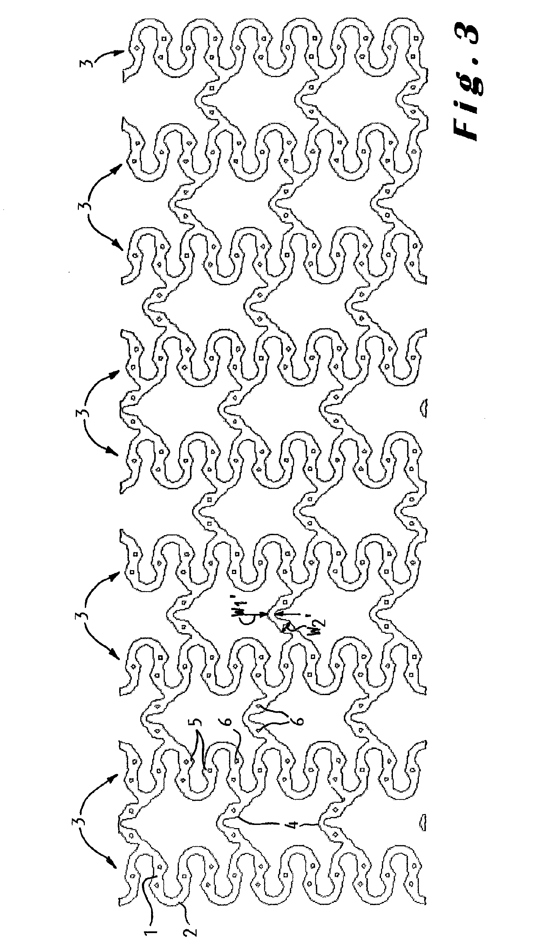

Figure 3 illustrates another example of the prosthesis according to the invention. In this example, the successive longitudinal struts 1 show alternately one and two locations 5 which are provided with one hole 6, the locations 5 on each pair of successive longitudinal struts 1 being further longitudinally displaced with respect to one another. An important advantage of this embodiment is that in this way the minimum distance between two successive longitudinal struts 1 is increased resulting in an increased flexibility and/or crimpability of the prosthesis. Compared to the embodiment illustrated in Figures 1 and 2, the longitudinal struts of the embodiment of Figure 3 comprise less holes 6 but this smaller amount of holes is compensated nearly completely by providing on each V-shaped connecting strut 4 two holes 6. Since these holes 6 are provided on the straight portions of the connecting struts 4, the flexibility thereof is little affected. As mentioned already herebefore, these connecting struts 4 have a width W'₁ of about 100 µm. At the location of the holes 6, the connecting struts have also an increased width, more particularly an increased width W'₂ of about 250 µm.

The holes 6 are preferably situated substantially in the centre of the locations 5. Each location preferably contains one hole. When a strut contains more holes, in particular two or more holes, the strut shows preferably a corresponding number of locations with an increased width. In this way, the strut has between those locations a strut width which is smaller than the increased width at the locations of the holes so that especially the crimpability of the prosthesis is less affected by the presence of the widenings, at least when the widenings are staggered or displaced with respect to one another so that, when crimping the prosthesis to a smaller diameter, the widenings on one strut can engage within the space provided between or next to the widenings (or widining) on an opposite strut.

The widenings of the struts at the location of the holes 6 may show different shapes, in particular rounded shapes such as an elliptical or circular shape. In the embodiments illustrated in the drawings, the holes are in the centre of a circle having in particular a radius of about 125 pm, the corners between the sections of this circle extending outside the normal strut width and the basic strut portion being rounded off somewhat.

From the above given description of some particular embodiments of the prosthesis according to the invention, it will be clear that these embodiments can be modified in different ways without departing from the scope of the appended claims. The prosthesis can be made for example from different materials, in particular from stainless steel, nitinol, cobalt-chromium alloys or Sandvik Nanoflex TM and may show different designs. The prosthesis may further show varying dimensions depending on the size of the lumen wherein it is to be applied.

## Claims

1. A radially expandable prosthesis for implantation in a lumen, showing an inner and an outer surface and comprising a tubular wall composed of elongated struts with a predetermined strut width and arranged to enable an expansion from a non-expanded state of the prosthesis to an expanded state, the prosthesis having an outer surface provided with holes, **characterised in that** at least a number of said struts show at least one location which is provided with at least one of said holes and at which the strut has an increased width larger than the predetermined width of the strut before and/or after said location.

2. A prosthesis according to claim 1, **characterised in that** said increased width is at least 5%, preferably at least 20% and more preferably at least 50% larger than said predetermined strut width.

3. A prosthesis according to claim 1 or 2, **characterised in that** said hole is a perforating hole and has an average width measured over the thickness of the strut, in a direction perpendicular to the longitudinal direction of the strut, said increased width being at least equal to the sum of said strut width and said average hole width, and is preferably at least equal to the sum of said strut width and 1.5 times said averaged hole width.

4. A prosthesis according to any one of the claims 1 to 3, **characterised in that** said hole is a non-perforating hole showing a depth smaller than the thickness of the strut or a perforating hole showing a depth equal to the thickness of the strut, the hole having an average width measured over the depth of the hole, in a direction perpendicular to the longitudinal direction of the strut, which average hole width is larger than 10 µm, in particular larger that 20 µm and more particularly larger than 30 µm, but smaller than 130 µm, preferably smaller than 90 µm and most preferably smaller or equal to 60 µm.

5. A prosthesis according to any one of the claims 1 to 4, **characterised in that** said hole is a non-perforating hole showing a depth smaller than the thickness of the strut or a perforating hole showing a depth equal to the thickness of the strut, the hole having an average width measured over the depth of the hole, in a direction perpendicular to the longitudinal direction of the strut, which average width comprises at the most 70%, preferably at the most 60% of said strut width.

6. A prosthesis according to any one of the claims 1 to 5, **characterised in that** said hole is a non-perforating hole showing a depth smaller than the thickness of the strut or a perforating hole showing a depth equal to the thickness of the strut, the hole having an average width measured over the depth of the hole, in a direction perpendicular to the longitudinal direction of the strut, and an average length measured over the depth of the hole, in the longitudinal direction of the strut, which comprises at the most five times, preferably at the most three times, the average hole width, the average hole length being most preferably substantially equal to the average hole width.

7. A prosthesis according to any one of the claims 1 to 6, **characterised in that** said hole extend over a depth in the strut which is greater than 30%, preferably greater than 50% and most preferably greater than 60% of the thickness of the strut.

8. A prosthesis according to any one of the claims 1 to 7, **characterised in that** it comprises at least two mutually connected circumferential sets of struts each comprising an alternating succession of longitudinal struts, extending in a general longitudinal direction, and transverse struts, extending in a generally circumferential direction and interconnecting two successive longitudinal struts, at least a number of said longitudinal struts showing at least one of said locations provided with at least one hole.

9. A prosthesis according to claim 8, **characterised in that** at least a number of said longitudinal struts show at least two of said locations, the struts having between the two locations a strut width which is smaller than said increased width.

10. A prosthesis according to claim 8 or 9, **characterised in that** a number of successive longitudinal struts show at least one of said locations provided with at least one hole, the locations on each pair of successive longitudinal struts of said number of successive longitudinal struts being longitudinally displaced with respect to one another.

11. A prosthesis according to any one of the claims 8 to 10, **characterised in that** a number of successive longitudinal struts show alternately one and two of said locations provided with a hole.

12. A prosthesis according to any one of the claims 8 to 11, **characterised in that** said transverse struts are free of said locations.

13. A prosthesis according to any one of the claims 8 to 12, **characterised in that** said transverse struts are curved preferably over an angle of at least 120°, and more preferably over an angle of at least 140°.

14. A prosthesis according to any one of the claims 8 to 13, **characterised in that** said circumferential sets of struts are mutually connected by one or more longitudinal connecting struts, preferably undulating longitudinal connecting struts, at least a number of the longitudinal connecting struts showing preferably at least one of said locations provided with at least one hole.

15. A prosthesis according to any one of the claims 1 to 14, **characterised in that** at least a number of said locations, and preferably all of them, are provided with only one of said holes.

16. A prosthesis according to any one of the claims 1 to 15, **characterised in that** at least a number of said struts which show at least one of said locations have such a thickness that the ratio of the strut width before and/or after said locations over the strut thickness is greater than 0.5, and preferably greater than 0.6.
